# EUROPEAN PATENT SPECIFICATION

(11) **EP 3 229 898 B1**
(45) Date of publication and mention of the grant of the patent: **12.02.2020**
(21) Application number: 15820619.3
(22) Date of filing: 07.12.2015
(51) Int. Cl.: A61N 1/39

(54) **EMERGENCY MEDICAL DEVICE POSITIONABLE AT TWO DIFFERENT VIEWING ANGLES**
MEDIZINISCHE, IN ZWEI VERSCHIEDENEN BETRACHTUNGSWINKELN POSITIONIERBARE NOTFALLVORRICHTUNG
DISPOSITIF MÉDICAL D'URGENCE POSITIONNABLE À DEUX ANGLES DE VISION DIFFÉRENTS

(30) Priority: 10.12.2014 US 201462090049 P
(43) Date of publication of application: 18.10.2017
(73) Proprietor: Koninklijke Philips N.V., 5656 AE Eindhoven (NL)
(72) Inventor: GUINEY, Patrick, 5656 AE Eindhoven (NL); HEATH, Stephen Robert, 5656 AE Eindhoven (NL); CASWELL, John Bench, 5656 AE Eindhoven (NL)
(74) Representative: de Haan, Poul Erik
(86) International application number: PCT/IB2015/059395
(87) International publication number: WO 2016/092446

(56) References cited:
- US-A- 5 935 152
- US-A1- 2003 167 074
- US-A1- 2004 122 476
- US-A1- 2007 270 909

## Description

### BACKGROUND:

### Technical Field

This disclosure relates to medical instruments and more particularly to a treatment device with a touch screen including multiple pre-set angle positions for usage in different positions.

### Description of the Related Art

Heart monitors and/or defibrillators typically include mechanical control knobs, buttons and switches. With the current generation of products, the user directly controls power and adjusts settings by manually turning the device on and off, and adjusting knobs and buttons to achieve desired settings. Newer defibrillator / monitor devices include advanced features and LCD displays but lack the sophistication and flexibility of modern interfaces, such as touchscreens, or other advanced devices.

Monitor/defibrillator screens are typically oriented at 60° to 90° relative to the horizontal plane or resting surface. Medics arriving at accident scenes where patients collapse or are otherwise on the ground must position the monitor defibrillator next to a patient on the floor or ground. While treating the patient from a kneeling position, medics must also operate and manipulate the monitor/defibrillator.

Operating a device positioned at 60° to 90° relative to the floor or ground when kneeling on the floor or ground requires an awkward wrist posture. When using a touchscreen, the operator must visualize the display while positioning their hand below the target touch zone and then bend the wrist upward to touch the desired button. The hazards of increasing the wrist angle of bending are well documented in medical literature. Repetitive, upward bending of the wrist causes irritation of tendons and tendon sheaths and will lead to a variety of health consequences including carpal tunnel syndrome. The angle of 60° to 90° relative to a horizontal surface is more appropriate for operating the monitor/defibrillator from a seated or standing posture when transporting a patient in an ambulance or on a gurney or when the resting surface is a countertop / shelf in an ambulance or on a gurney. The document US 5 935 152 A discloses a portable defibrillator which can hang from a first support member and rest against a second support member.

### SUMMARY

In accordance with the present principles, an emergency medical device includes a treatment device including a display face, a base including a bottom portion configured to rest on a resting surface, and a back portion configured to extend beyond a rear surface of the treatment device opposite the front portion. A rear structure is connected to the treatment device and extends beyond the rear surface of the treatment device wherein the base is configured to provide a first angle of the display face when the bottom portion is resting on the resting surface, and wherein the back portion and the rear structure provide a second angle of the display face when the back portion and the rear structure are resting on the resting surface.

Another emergency medical device includes a base coupled to a treatment device having a display face. The base includes a front portion, a bottom portion configured to rest on a resting surface and a back portion configured to extend beyond a rear surface of the treatment device opposite the front portion. A rear bracket is connected to the treatment device and extends beyond the rear surface of the treatment device. The base is configured so that the front portion has a different offset dimension from the treatment device than the back portion to provide a first angle of the display face when the bottom portion is resting on the resting surface. The back portion and the rear bracket provide a second angle of the display face when the back portion and the rear bracket are resting on the resting surface.

Yet another emergency medical device includes a treatment device having a touchscreen display positioned on a face thereof. A base is coupled to the treatment device and includes a front portion, a bottom portion configured to rest on a resting surface and a back portion configured to extend beyond a rear surface of the treatment device opposite the face of the treatment device. At least one rear bracket is connected to the treatment device and extends beyond the rear surface of the treatment device wherein the base is configured to provide at least two viewing positions for the touchscreen display such that the front portion has a different offset dimension from the treatment device than the back portion to provide a first angle of the touchscreen display when the bottom portion is resting on the resting surface, and wherein the back portion and the at least one rear bracket provide a second angle of the touchscreen display when the back portion and the at least one rear bracket are resting on the resting surface. A handle is disposed on the treatment device on a side opposite that of the base, the handle permitting a user to adjust the device between the at least two viewing positions.

A method for positioning an emergency medical device includes providing a treatment device with a display face, a base coupled to the treatment device, the base including a front portion, a bottom portion configured to rest on a resting surface and a back portion configured to extend beyond a rear surface of the treatment device opposite the front portion; at least one rear bracket connected to the treatment device and extending beyond the rear surface of the treatment device wherein the base is configured so that the front portion has a different offset dimension from the treatment device than the back portion to provide a first angle of the display face when the bottom portion is resting on the resting surface, and wherein the back portion and the at least one rear bracket provide a second angle of the display face when the back portion and the at least one rear bracket are resting on the resting surface; and changing positions of the device between the first and second angles relative to resting surfaces in accordance with an operator position.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a front view showing a defibrillator/monitor device with a base and a handle in accordance with one embodiment;
FIG. 2 is a side view showing the defibrillator/monitor device of FIG. 1 with the base in contact with a resting surface to provide a first preset angle in accordance with one embodiment;
FIG. 3 is a side view showing the defibrillator/monitor device of FIG. 2 with the base and a rear bracket in contact with a resting surface to provide a second preset angle in accordance with one embodiment;
FIG. 4 is a bottom view showing the defibrillator/monitor device of FIG. 1 with a U-shaped base in accordance with one embodiment; and
FIG. 5 is a block/flow diagram showing a method for operating the defibrillator/monitor in different positions.

### DETAILED DESCRIPTION OF EMBODIMENTS

In accordance with the present principles, monitors and/or defibrillators may be provided with a touchscreen interface. The operation of a touchscreen on a monitor/defibrillator in accordance with the present principles employs more than one angle of the screen relative to a resting surface to accommodate multiple postures of medics in different care settings. The monitor/defibrillator includes geometry with mechanical features that permit users to position the touchscreen at more than one angle. For example, one angle of the display may appropriately position the touchscreen for an operator kneeling on the ground or floor. This is particularly useful where medics and first responders provide care from a kneeling position at accident scenes where patients collapse. A second angle may be employed to position the touchscreen for a seated or standing operator. During transport, medics may sit or stand in the back of an ambulance while caring for the patient. A monitor/defibrillator with features for multi-angle positioning meets the needs of medics that operate the user interface from more than one position or posture.

In particularly useful cases, operators utilize the touch screens to interact with the devices using single-touch controls. Menus and soft keys are activated using a single touch by the user on the touch screen. Multi-touch controls may also be employed for zooming, paging, scrolling, and other display navigation functions accomplished by having the user pinch and expand or touch rand swipe the touch screen. A graphical user interface in accordance with the present principles utilizing touch screen technology provides both single-touch and multi-touch controls. By providing preset stable angle positioning, touchscreen operations are easily carried out in multiple positions in an ergonomic manner (e.g., without back bending of the wrists.)

Devices in accordance with the present principles have mechanical features and geometry to permit users to position a display at more than one angle to accommodate multiple postures of caregivers in different care settings. The design geometry and features are simple and passive with no mechanisms, latches or moving parts to malfunction. The mechanical design allows fast operation with one hand in time critical, urgent care settings.

It should be understood that the present apparatus will be described in terms of medical instruments; however, the teachings and are applicable to any monitor device or defibrillator operated in different positions. In some cases, the present principles are employed in defibrillators/monitors used in hospitals or emergency vehicles, in homes, in public places. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor or controller within a treatment device, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage.

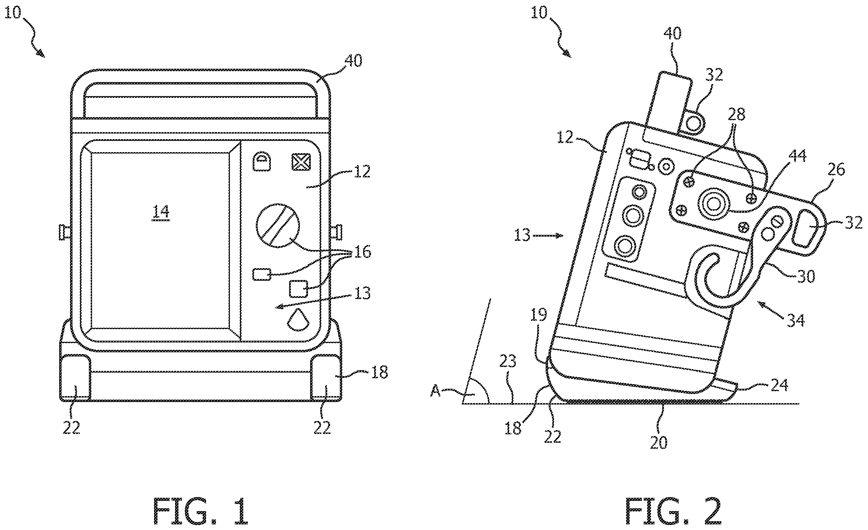

Referring now to the drawings in which like numerals represent the same or similar elements and initially to FIGS. 1 and 2, a diagram shows an emergency medical apparatus or device 10, which includes a treatment device 12, such as a defibrillator, in accordance with one embodiment. Device 12 includes a plurality of controls 16 and a touch screen interactive display 14 on a display face 13. Apparatus 10 or device 12 includes one or more mechanical features that that are attachable externally to the device 12 or may be integrated into the device 12.

In one case, the mechanical features may include a base 18 connectable to the device 12 below the display 14. The base 18 includes a front portion 22 that includes connection points 19 to a front of the device 12. The connection points 19 may include snap-fit mechanisms, screw holes, rivets, magnets or other connection mechanisms to hold the front portion 22 to the device 12. The base 18 may be permanently affixed to or temporarily mounted to the device 12. The base 18 provides a larger support height at the front portion 22 than a support height of a back portion 24. In this way, the base 18 supports device 12 in an upright configuration at an angle "A" preferably between about 45 degrees to less than about 90 degrees with respect to a resting surface 23. In one case, the angle A is even more preferably between about 60 degrees and 80 degrees. A bottom portion 20 rests on the resting surface 23 and may include grips, treads or other mechanical features that prevent slippage, reduce or absorb shock. The base 18 may include a combination of materials including rubber, plastics, metals, ceramics. In one case, at least the bottom portion 20 includes a rubber or rubberized surface.

In one case, the mechanical design features on the base 18 of the device 12 position the touchscreen 14 to a 75° angle relative to a resting surface. The mechanical features or feet of the base 18 provide a stable base to support the entire weight of the device 12 from front to rear on each side and across a back edge or back portion 24 of the bottom portion 20. FIG. 2 shows a side view of the bottom portion 24 on the resting surface 23 to achieve the desired touchscreen angle of 75°. The bottom portion 20 may be textured to prevent slippage on smooth surfaces and is preferably made from durable, compliant material to help prevent damage from drop shock.

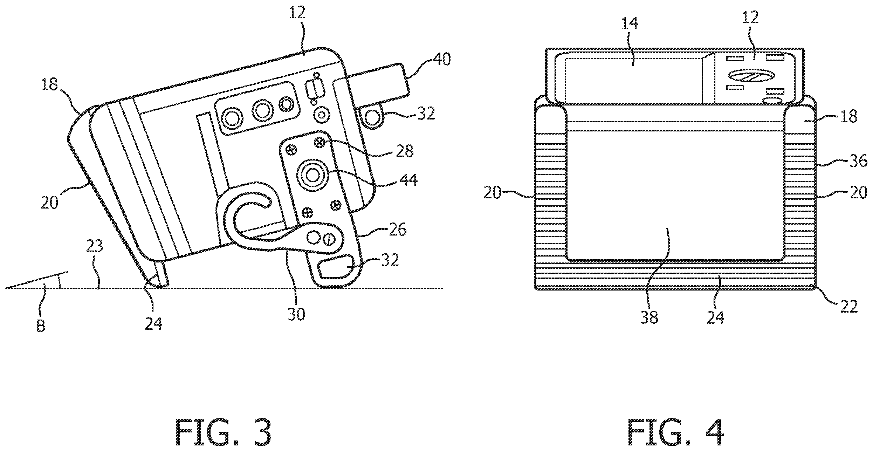

The position of the touch screen 14 in FIG. 2 provides an improved operational position while an operator is seated or standing. An angle A between about 60° and 90° is preferred in such positions. FIG. 2 also depicts a rear bracket 26 affixed to a side (or back surface) of the device 12. There may be a pair of rear brackets 26, one on each side of the device 12 that may be bolted, riveted, snapped or otherwise attached to the device 12. The rear brackets 26 may be connected to one another across the rear of the treatment device 12. The rear brackets 26 may take on any suitable configuration or structure. The rear brackets 26 may include a shock absorbing, weight bearing material to permit the device to be rotated backward to support the device 12 in a position shown in FIG. 3.

Referring to FIG. 3, the device 12 is depicted in a second position in which the rear brackets 26 and the back portion 24 are in contact with a resting surface. An angle position B for touch screen operation while an operator is in a kneeling position is preferably between about 0° to about 30°. In one embodiment, mechanical design features (the rear brackets 26 and the back portion 24) at the rear of the device 12, position the touchscreen at a 15° angle relative to the resting surface. The first position described in FIG. 2 is provided based upon a shape of the base 18 whereas the second position in FIG. 3 is provided based upon the rear brackets 26 and the back portion 24 of the base 18. The same bottom surface features (24) (feet) described above that extend slightly beyond the rear edge of the device 12 are advantageously employed. The rear brackets 26 also provide attachment points for rail hooks 30, shoulder strap eyes 32. These can be located at multiple locations. A space 34 can be provided for rear storage or a storage pouch. Together, the rear brackets 26 of the back portion 24 form a stable support to achieve the desired 15° angle (or other angle) for operating the device from a kneeling or squatting position of the operator.

The base 18 and/or brackets 26 may include materials, such as plastic, metal, elastomers and fabrication methods may include, e.g., injection molding, machining. A top handle 40 allows medics or operators to use a single hand to grasp the device top and push back the device to the low angle position of operation (0° to 30°) while kneeling. Alternatively, by grasping the handle 40 and pulling forward, the medic can return the device 12 to the high angle position of operation (60° to 90°) for the operation while seated/standing. In one case, the rear brackets 26 may be rotatably adjusted to change the second position angle B. In this case, screws 28 may be removed or not included and a pivot 44 may be adjusted to determine a new angle for the second position. In other cases, the base 18 may be configured to provide an adjustment of the first position using a thumb screw or other adjustment mechanism to change the angle A. In yet other cases, other brackets or adjustments may be available to provide additional adjustment or to provide more than two set positions for the apparatus 10, for example, further extending or retracting brackets 26 or the back portion 24 of the base 18.

Referring to FIG. 4, a bottom view of the apparatus 10 is illustratively shown in accordance with one embodiment. In this embodiment, the bottom portion 20 of the base 18 includes a U shape following along sides and back of the apparatus 10. The bottom portion 20 includes grip features, treads 36 or textures to prevent slippage. In some embodiments, a space 38 may include other structures or base features that may connect to the device 12 and provide support, dampening or additional structures.

While the present principles are described in terms of a monitor/defibrillator, two types of monitor defibrillators, in particular, can benefit from multi-angle positioning as described herein. These include in-hospital monitor defibrillators (used by hospital personnel) and pre-hospital monitor defibrillators (used by emergency medical services (EMS), public safety, military personnel). Other medical devices used in care settings that call for different operating postures may also benefit from the present principles.

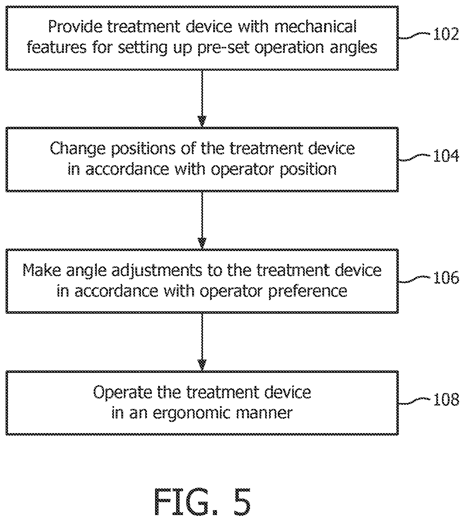

Referring to FIG. 5, a method for positioning an emergency medical device is illustratively shown in accordance with the present principles. In block 102, a treatment device with a display face is provided. The treatment device preferably includes a touchscreen display on the display face, and mechanical features for setting stable preset angle positions for the display. The treatment device may be integrated with or be retrofitted with a base and at least one rear bracket. The base is coupled (formed or connected) to the treatment device and includes a front portion, a bottom portion configured to rest on a resting surface and a back portion configured to extend beyond a rear surface of the treatment device opposite the front portion. The rear bracket or brackets is/are connected to the treatment device and extend beyond the rear surface of the treatment device. The base is configured so that the front portion has a different offset dimension from the treatment device than the back portion to provide a first angle of the display face when the bottom portion is resting on the resting surface. The back portion and the rear bracket(s) provide a second angle of the display face when the back portion and the at least one rear bracket are resting on the resting surface.

In block 104, the device can have its positions changed, e.g., between the first and second angles relative to a resting surface and in accordance with an operator position. The treatment device includes the touchscreen display positioned on the display face and the first angle and the second angle permit ergonomic touchscreen operation by a user in different positions. For example, at the first angle, e.g., between about 60 degrees and about 90 degrees with the resting surface, the operator may be sitting or standing. At the second angle between about 0 degrees and about 30 degrees with the resting surface, the operator may be kneeling or squatting. The device is advantageously tilted back or forward to provide a preset display angle that is optimized for the position of the operator.

The device may include more than two present angle positions by providing other mechanical features to support additional stable positions of the device. In block 106, adjustments may be made to the base and/or the rear brackets to adjust the first and/or second angles. This may be made based on operator preference or other considerations.

In block 108, the device is operated in an ergonomic manner using the touchscreen display. Since the touch screen display is appropriately angled based on the user's position, back bending of the wrist and other arm or wrist discomfort are avoided. The invention is defined by the appended claims.

## Claims

1. An emergency medical device, comprising:
a treatment device (12) including:
a display face (13);
a base (18) including a bottom portion (20) configured to rest on a horizontal resting surface, and a back portion (24) configured to extend beyond a rear surface of the rest of the treatment device opposite the front portion; and
a rear structure (26) connected to the treatment device and extending beyond the rear surface of the treatment device, the emergency medical device rotatable to change an angle of the display face from a first angle with the resting surface when the bottom portion of the base is resting on the resting surface to a second angle with the resting surface when the back portion and the rear structure are resting on the resting surface, **characterized in that** the back portion is the back edge of the bottom portion.

2. The device as recited in claim 1, wherein the treatment device includes a touchscreen display (14) positioned on the display face of the treatment device.

3. The device as recited in claim 1, wherein the bottom portion include treads (36) to prevent slippage relative to the resting surface.

4. The device as recited in claim 1, wherein the base (18) includes a U-shaped bottom portion extending along sides and the rear surface of the rest of the treatment device.

5. The device as recited in claim 1, wherein the rear structure (26) includes at least one rear bracket (26) affixed to a side or sides of the treatment device.

6. The device as recited in claim 5, wherein the at least one rear bracket (26) includes a support accessory including one or more of: a support hook and a support eye.

7. The device as recited in claim 1, wherein the first angle is between about 60 degrees and about 90 degrees with the resting surface.

8. The device as recited in claim 1, wherein the second angle is between about 0 degrees and about 30 degrees with the resting surface.

9. The device as recited in claim 1, wherein the treatment device (12) includes one of a monitor, a defibrillator or a combination thereof.

10. The device as recited in claim 1, wherein the display is a touchscreen display (14); wherein the rear structure comprises at least one rear bracket (26); wherein the base is configured to provide at least two viewing positions for the touchscreen display such that the front portion has a different offset dimension from the treatment device than the back portion to provide a first angle of the touchscreen display when the bottom portion is resting on the resting surface, and wherein the back portion and the at least one rear bracket provide a second angle of the touchscreen display when the back portion and the at least one rear bracket are resting on the resting surface; and wherein the device further comprises a handle (40) disposed on the treatment device on a side opposite that of the base, the handle permitting a user to adjust the device between the at least two viewing positions.

11. The device as recited in claim 10, wherein the bottom portion (20) includes treads (36) to prevent slippage relative to the resting surface.

12. The device as recited in claim 10, wherein the at least one rear bracket (26) includes rear brackets affixed to opposite sides of the treatment device.

13. The device as recited in claim 10, wherein the at least one rear bracket (26) includes a support accessory including one or more of: a support hook and a support eye.

14. The device as recited in claim 10, wherein a first angle is between about 60 degrees and about 90 degrees with the resting surface.

15. The device as recited in claim 10, wherein a second angle is between about 0 degrees and about 30 degrees with the resting surface.

## Patentansprüche

1. Medizinische Notfallvorrichtung, umfassend:
eine Behandlungsvorrichtung (12), beinhaltend:
eine Anzeigefläche (13);
einen Sockel (18), der einen unteren Abschnitt (20) beinhaltet, der konfiguriert ist, um auf einer horizontalen Auflageoberfläche aufzuliegen, und einen hinteren Abschnitt (24), der konfiguriert ist, um sich über eine rückwärtige Oberfläche der Auflage der Behandlungsvorrichtung gegenüber dem vorderen Abschnitt zu erstrecken; und
eine rückwärtige Struktur (26), die mit der Behandlungsvorrichtung verbunden ist und sich über die rückwärtige Oberfläche der Behandlungsvorrichtung hinaus erstreckt, wobei die medizinische Notfallvorrichtung drehbar ist, um einen Winkel der Anzeigefläche von einem ersten Winkel zu der Auflageoberfläche, wenn der untere Abschnitt des Sockels auf der Auflageoberfläche aufliegt, in einen zweiten Winkel zu der Auflageoberfläche, wenn der hintere Abschnitt und die rückwärtige Struktur auf der Auflageoberfläche aufliegen, zu verändern, **dadurch gekennzeichnet, dass** sich der hintere Abschnitt in der hinteren Kante des unteren Abschnitts befindet.

2. Vorrichtung nach Anspruch 1, wobei die Behandlungsvorrichtung eine Touchscreen-Anzeige (14) beinhaltet, die auf der Anzeigefläche der Behandlungsvorrichtung positioniert ist.

3. Vorrichtung nach Anspruch 1, wobei der untere Abschnitt Profile (36) beinhaltet, um ein Rutschen in Bezug auf die Auflageoberfläche zu verhindern.

4. Vorrichtung nach Anspruch 1, wobei der Sockel (18) einen U-förmigen unteren Abschnitt beinhaltet, der sich entlang Seiten und der rückwärtigen Oberfläche der Auflage der Behandlungsvorrichtung erstreckt.

5. Vorrichtung nach Anspruch 1, wobei die rückwärtige Struktur (26) mindestens eine rückwärtige Halterung (26) beinhaltet, die an einer Seite oder Seiten der Behandlungsvorrichtung befestigt ist.

6. Vorrichtung nach Anspruch 5, wobei die mindestens eine rückwärtige Halterung (26) ein Tragezubehörteil beinhaltet, das eines oder mehrere von folgenden beinhaltet: einen Tragehaken und eine Trageöse.

7. Vorrichtung nach Anspruch 1, wobei der erste Winkel zwischen etwa 60 Grad und etwa 90 Grad zu der Auflageoberfläche beträgt.

8. Vorrichtung nach Anspruch 1, wobei der zweite Winkel zwischen etwa 0 Grad und etwa 30 Grad zu der Auflageoberfläche beträgt.

9. Vorrichtung nach Anspruch 1, wobei die Behandlungsvorrichtung (12) einen von einem Monitor, einem Defibrillator oder einer Kombination davon beinhaltet.

10. Vorrichtung nach Anspruch 1, wobei die Anzeige eine Touchscreen-Anzeige (14) ist;
wobei die rückwärtige Struktur mindestens eine rückwärtige Halterung (26) umfasst;
wobei der Sockel konfiguriert ist, um mindestens zwei Ansichtspositionen für die Touchscreen-Anzeige bereitzustellen, sodass der vordere Abschnitt eine andere versetzte Abmessung von der Behandlungsvorrichtung aufweist als der hintere Abschnitt, um einen ersten Winkel der Touchscreen-Anzeige, wenn der untere Abschnitt auf der Auflageoberfläche aufliegt, bereitzustellen, und wobei der hintere Abschnitt und die mindestens eine rückwärtige Halterung auf der Auflageoberfläche aufliegen; und
wobei die Vorrichtung weiter Folgendes umfasst:
einen Griff (40), der auf der Behandlungsvorrichtung auf einer Seite gegenüber der des Sockels angeordnet ist, wobei der Griff es einem Benutzer erlaubt, die Vorrichtung zwischen den mindestens zwei Ansichtspositionen einzustellen.

11. Vorrichtung nach Anspruch 10, wobei der untere Abschnitt (20) Profile (36) beinhaltet, um ein Rutschen in Bezug auf die Auflageoberfläche zu verhindern.

12. Vorrichtung nach Anspruch 10, wobei die mindestens eine rückwärtige Halterung (26) rückwärtige Halterungen beinhaltet, die an gegenüberliegenden Seiten der Behandlungsvorrichtung befestigt sind.

13. Vorrichtung nach Anspruch 10, wobei die mindestens eine rückwärtige Halterung (26) ein Tragezubehörteil beinhaltet, das eines oder mehrere von folgenden beinhaltet: einen Tragehaken und eine Trageöse.

14. Vorrichtung nach Anspruch 10, wobei ein erster Winkel zwischen etwa 60 Grad und etwa 90 Grad zu der Auflageoberfläche beträgt.

15. Vorrichtung nach Anspruch 10, wobei ein zweiter Winkel zwischen etwa 0 Grad und etwa 30 Grad zu der Auflageoberfläche beträgt.

## Revendications

1. Dispositif médical d'urgence comprenant :
un dispositif de traitement (12) incluant :
une face d'affichage (13) ;
une base (18) incluant une partie inférieure (20) configurée pour reposer sur une surface de repos horizontale et une partie de dos (24) configurée pour s'étendre au-delà d'une surface arrière du reste du dispositif de traitement opposé à la partie avant ; et
une structure arrière (26) raccordée au dispositif de traitement et s'étendant au-delà de la surface arrière du dispositif de traitement, le service médical d'urgence pouvant tourner pour changer un angle de la face d'affichage d'un premier angle avec la surface de repos lorsque la partie inférieure de la base repose sur la surface de repos à un second angle avec la surface de repos lorsque la partie de dos et la structure arrière reposent sur la surface de repos, **caractérisé en ce que** la partie de dos est le bord de dos de la partie inférieure.

2. Dispositif selon la revendication 1, dans lequel le dispositif de traitement inclut un affichage d'écran tactile (14) positionné sur la face d'affichage du dispositif de traitement.

3. Dispositif selon la revendication 1, dans lequel la partie inférieure inclut des sculptures (36) pour empêcher le patinage par rapport à la surface de repos.

4. Dispositif selon la revendication 1, dans lequel la base (18) inclut une partie inférieure en forme de U s'étendant le long des côtés et de la surface arrière du reste du dispositif de traitement.

5. Dispositif selon la revendication 1, dans lequel la structure arrière (26) inclut au moins un support arrière (26) fixé à un ou des côtés du dispositif de traitement.

6. Dispositif selon la revendication 5, dans lequel le au moins un support arrière (26) inclut un accessoire de support incluant un ou plusieurs d'un crochet de support et d'un œillet de support.

7. Dispositif selon la revendication 1, dans lequel le premier angle se situe entre environ 60 degrés et environ 90 degrés avec la surface de repos.

8. Dispositif selon la revendication 1, dans lequel le second angle se situe entre environ 0 degré et environ 30 degrés avec la surface de repos.

9. Dispositif selon la revendication 1, dans lequel le dispositif de traitement (12) inclut un élément parmi un moniteur, un défibrillateur ou une de leurs combinaisons.

10. Dispositif selon la revendication 1, dans lequel l'affichage est un affichage d'écran tactile (14) ;
dans lequel la structure arrière comprend au moins un support arrière (26) ;
dans lequel la base est configurée pour fournir au moins deux positions d'observation pour l'affichage d'écran tactile de sorte que la partie avant ait une dimension décalée différente du dispositif de traitement que de la partie de dos pour fournir un premier angle de l'affichage d'écran tactile lorsque la partie inférieure repose sur la surface de repos et dans lequel la partie de dos et le au moins un support arrière fournissent un second angle de l'affichage à écran tactile lorsque la partie de dos et le au moins un support arrière reposent sur la surface de repos ; et dans lequel le dispositif comprend en outre :
une poignée (40) disposée sur le dispositif de traitement sur un côté opposé à celui de la base, la poignée permettant à un utilisateur d'ajuster le dispositif entre les au moins deux positions d'observation.

11. Dispositif selon la revendication 10, dans lequel la partie inférieure (20) inclut des sculptures (36) pour empêcher un patinage par rapport à la surface de repos.

12. Dispositif selon la revendication 10, dans lequel le au moins un support arrière (26) inclut des supports arrière fixés aux côtés opposés du dispositif de traitement.

13. Dispositif selon la revendication 10, dans lequel le au moins un support arrière (26) inclut un accessoire de support incluant un ou plusieurs d'un crochet de support et d'un œillet de support.

14. Dispositif selon la revendication 10, dans lequel un premier angle est compris entre environ 60 degrés et environ 90 degrés avec la surface de repos.

15. Dispositif selon la revendication 10, dans lequel un second angle est compris entre environ 0 degré et environ 30 degrés avec la surface de repos.
